**Europäisches Patentamt**

(19) **European Patent Office**

**Office européen des brevets**

(11) Numéro de publication: **0 005 653**
**B1**

(12) **FASCICULE DE BREVET EUROPEEN**

(45) Date de publication du fascicule du brevet: **28.07.82**

(51) Int. Cl.³: **C 07 J 9/00**

(21) Numéro de dépôt: **79400130.5**

(22) Date de dépôt: **01.03.79**

(54) **Procédé de préparation de dihydroxy-24,25-cholestérol à partir du desmostérol.**

(30) Priorité: **11.03.78 OA 56433**

(43) Date de publication de la demande:
**28.11.79 Bulletin 79/24**

(45) Mention de la délivrance du brevet:
**28.07.82 Bulletin 82/30**

(84) Etats contractants désignés:
**BE CH DE FR GB**

(56) Documents cités:
**US - A - 3 928 397**

**CHEMICAL ABSTRACTS, vol. 80, n° 23, 10 juin 1974 Columbus, Ohio, USA—JOSEPH REDEL et al: "Synthesis of 24, 25 dihydroxycholecalciferol 1 polar metabolites of vitamin D3" page 504, 2ème colonne, abrégé 133685a**
**TETRAHEDRON LETTERS, novembre 1973, n° 45 Oxford GB**
**L. MANGONI et la.: "A convenient procedure for the as-hydroxylation of olefins", pages 4485—4486**

(73) Titulaire: **ETAT DU CAMEROUN représenté par le Ministère de l'Education Nationale Yaounde (CM)**

(72) Inventeur: **Tchienehom, René décédé domicilié préalablement à: Bayancam (CM)**
Inventeur: **Charles, Georges Les Goumoines route de Bouffard F-17390 La Tremblade (FR)**

(74) Mandataire: **de Haas, Michel et al, Cabinet Beau de Loménie 55 rue d'Amsterdam F-75008 Paris (FR)**

## Procédé de préparation de dihydroxy-24,25 cholestérol à partir du desmostérol

La présente invention a pour objet des procédés pour l'obtention du dihydroxy-24,25 cholestérol à partir du desmostérol soit pur, soit en mélange avec d'autres stérols naturels.

L'invention concerne également la préparation, en tant que stéroide intermédiaire, de diacétoxy-3β,24 hydroxy-25 cholestène-5.

Le composé ci-dessus est connu comme produit de base pour la synthèse de la dihydroxy-24,25 vitamine $D_3$ et de ses dérivés. Son importance a été décrite par divers auteurs et principalement les suivants: Masao Seki, Julieta Rubio-Lightbourn, Masuo Morisaki et Nobuo Ikekawa (Chem. Pharm. Bull. 1973, 21, 2783—2785); Joseph Redel, Philip Bell, Florian Delbarre et Egon Kodicek (C. R. Acad. Sc. de Paris 1974, 278, série D, 529—531); Masao Seki, Naoyuki Koizumi, Masuo Morisaki et Nobuo Ikekawa (Tetrahedron Letters 1975, 1, 15—18). Il est aussi connu comme produit de base pour la synthèse d'hormones du type ecdysone.

La présente invention permet d'obtenir ce composé intermédiaire en mettant en oeuvre un procédé utilisant une réaction simple et directe. En effet, la transformation du desmostérol, ou du mélange avec d'autres stérols naturels le contenant, en diacétoxy-3β,24 hydroxy-25 cholestène-5 se fait par exemple, en une seule étape, avec des réactifs courants tels que l'iode ($I_2$), l'iodate de potassium ($KIO_3$), l'acétate de potassium ($CH_3COOK$) et l'acide acétique ($CH_3COOH$). La réaction ci-dessus se conduit aussi bien à partir du desmostérol pur qu'à partir d'un mélange de stérols naturels le contenant. La mise en oeuvre du procédé de l'invention à partir du mélange de stérols naturels permet d'éviter le recours aux procédés longs et complexes d'obtention du desmostérol pur.

Le dihydroxy-24,25 cholestérol est ensuite très facilement obtenu par hydrolyse du diacétoxy-3β,25 hydroxy-25 cholestène-5.

Le brevet DAPI n° 4246 de Thomas NJIMI et Georges CHARLES donne des procédés d'obtention des produits de base de cette préparation, c'est-à-dire le desmostérol ou le mélange de stérols le contenant, à partir de graines de Funtumia elastica, mais également à partir d'autres parties de cette plante ou à partir d'autres plantes de l'embranchement des Phanérogames et, en particulier, la famille des Apocynacées tels que les genres Funtumia ou Holarrhéna. Dans la présente description, les exemples sont donnés avec des produits de départ provenant des graines de Funtumia elastica selon la description dudit brevet.

Le processus de mise en oeuvre de la présente invention comporte un schéma principal avec deux variantes. Ces procédés consistent foundamentalement à transformer le desmostérol, pur ou en mélange avec d'autres stérols naturels, en diacétoxy-24 hydroxy-25 cholestène-5 en le traitant dans l'acide acétique par l'iode, l'iodate de potassium et, éventuellement, par l'acétate de potassium (cf. L. Mangoni, M. Adinolfi, G. Barone et M. Parrilli, Tetrahedron Letters 1973, 45, 4485—4486).

Dans le procédé de base, le desmostérol ou le mélange de stérols naturels en contenant est d'abord acétylé, par exemple par l'anhydride acétique dans la pyridine, puis traité dans l'acide acétique par l'iode $I_2$ en présence d'iodate de potassium $KIO_3$ en suivant l'avancement de la réaction par des chromatographies sur couche mince. Lorsque la réaction est assez avancée, le mélange réactionnel est traité par l'acétate de potassium $CH_3COOK$. Quand la réaction est terminée, on ajoute une solution aqueuse de thiosulfate de sodium pour détruire l'iode en excès et le produit est extrait par un solvant, de préférence l'éther diéthylique. L'extrait organique obtenu est lavé par une solution de base, de préférence le bicarbonate de sodium jusqu'à l'arrêt de l'effervescence, puis à l'eau jusqu'à pH neutre des eaux de lavage. La solution organique est alors séchée sur un déshydratant comme le sulfate de sodium, filtrée et évaporée à sec sur bain-marie. Le résidu ($R_1$) obtenu est chromatographié sur colonne (de silice, d'alumine ou autre) en utilisant par exemple l'hexane auquel on ajoute progressivement des quantités croissantes d'éther diéthylique comme éluant. Le composé le plus polaire obtenu a été identifié comme diacétoxy-3β,24 hydroxy-25 cholestène-5. Ce dernier est ensuite hydrolysé en dihydroxy-24,25 cholestérol, par exemple suivant le protocole expérimental ci-après: le diacétoxy-3β,24 hydroxy-25 cholestène-5 est traité par une solution alcoolique de base (KOH, NaOH). Lorsque la réaction est terminée, on ajoute de l'eau et on extrait le produit par un solvant, de préférence le chloroforme. L'extrait organique obtenu est lavé par une solution d'acide, de préférence l'acide chlorhydrique, puis à l'eau. La solution organique est alors séchée sur un déshydratant comme le sulfate de sodium, filtrée et évaporée à sec. Le dihydroxy-24,25 cholestérol ainsi obtenu est recristallisé dans un solvant, de préférence l'acétone.

Selon le première variante, le desmostérol ou le mélange de stérols en contenant est d'abord acétylé, par exemple par l'anhydride acétique dans la pyridine, puis traité dans l'acide acétique par l'iode $I_2$ en présence d'iodate de potassium $KIO_3$ pendant un temps 2 à 3 fois plus long que la durée de la réaction décrite dans le procédé de base. Ce procédé est économique puisqu'on supprime l'emploi de l'acétate de potassium $CH_3COOK$. Le mélange réactionnel est ensuite traité suivant le processus décrit dans le procédé de base, à savoir addition de solution de thiosulfate de sodium, extraction du produit, lavages, séchage, filtration, évaporation à sec,

3 **0 005 653** 4

chromatographie sur colonne du résidu obtenu, hydrolyse (saponification) du diacétoxy-3β,24 hydroxy-25 cholestène-5 obtenu.

Selon la seconde variante, le résidu (R₁) obtenu selon le procédé de base ou le résidu (R₂) obtenu selon la variante 1 est séparé par cristallisation fractionnée dans un solvant, de préférence dans l'hexane. Ce procédé a pour avantage la suppression de la chromatographie sur colonne. On obtient du diacétoxy-3β,24 hydroxy-25 cholestène-5 pratiquement pur qui est ensuite hydrolysé (saponifié) selon le processus de base.

Le présente invention concerne donc un procédé de préparation du dihydroxy-24,25 cholestérol, caractérisé en ce que l'on soumet du desmostérol pur ou en mélange avec d'autres stérols naturels à une réaction d'acétylation, puis on traite le mélange réactionnel obtenu dans l'acide acétique par l'iode en présence d'iodate de potassium, puis l'iode en excès est détruit, que l'on extrait le produit réactionnel et évapore à sec la solution organique, que l'on soumet le résidu obtenu à une chromatographie ou à une cristallisation fractionnée et enfin que l'on hydrolyse le diacétoxy-3β,24 hydroxy-25 cholestène-5 par une solution alcoolique basique.

Pour une bonne compréhension de la présente invention, on donne ci-dessous, uniquement à titre d'exemples, quelques processus expérimentaux du procédé appliqué au desmostérol pur, puis au mélange de stérols provenant des graines de Funtumia elastica selon le brevet OAPI n° 4246.

Les exemples non limitatifs suivants illustrent l'invention.

Exemple 1

Le desmostérol est d'abord acétylé par l'anhydride acétique dans la pyridine. Ensuite, on dissout 500 mg d'acétyldesmostérol (1,17 mmole) dans 20 ml d'acide acétique pur cristallisable. On introduit un barreau aimanté et la solution est portée aux environs de 45°C à l'aide d'un bain-marie. On ajoute alore 126 mg d'iodate de potassium KIO₃ (0,59 mmole). Ensuite, par petites fractions et en agitant magnétiquement, on ajoute 150 mg d'iode I₂ (0,59 mmole) préalablement pulvérisé à l'aide d'un mortier et le mélange est maintenu autour de 45°C pendant 2 h sous agitation. On ajoute alore 118 mg d'acétate de potassium CH₃COOK (1,18 mmole) et le mélange est porté à 80°C en bain-marie pendant 2 h. Après refroidissement, on ajoute 60 ml d'une solution aqueuse de thiosulfate de sodium à 1% et le produit qui précipite est extrait trois fois à l'éther diéthylique. L'extrait éthéré est 1°) lavé par une solution aqueuse de bicarbonate de sodium à 20% jusqu'à l'arrêt de l'effervescence, 2°) lavé à l'eau jusqu'à pH neutre des eaux de lavage, 3°) séché sur sulfate de sodium, filtré et évaporé à sec sur bainmarie. Le résidu obtenu (625 mg) est séparé par chromatographie sur colonne de 20 g de silice. Le mélange hexane-éther éthylique: 99/1 élue l'acétyl-desmostérol de départ (25 mg). Le mélange hexane-éther éthylique: 99/1 élue l'acétyl-desmostérol de départ (25 mg). Le mélange hexane-éther éthylique: 85/15 élue le diacétoxy-3β,24 hydroxy-25 cholestène-5 (545 mg). Rendement:92%.

L'hydrolyse du diacétoxy-3β,24 hydroxy-25 cholestène-5 est effectuée de la façon suivante: 502 mg du diacétoxy-3β,24 hydroxy-25 cholestène-5 dissous dans 10 ml d'éthanol sont traités à reflux par 2 ml d'une solution de potasse à 25% dans l'éthanol à 85% pendant 1 h. Après refroidissement, on ajoute de l'eau et le produit est extrait plusieurs fois au chloroforme. L'extrait chloroformique obtenu est lavé par une solution d'acide chlorhydrique à 10% puis à l'eau. La solution organique est ensuite séchée sur sulfate de sodium, filtrée et évaporée à sec. Le résidu obtenu (400 mg) constitué du dihydroxy-24,25 cholestérol pratiquement pur est recristallisé dans l'acétone. Rendement: 96%.

Exemple 2

Le mélange de stérols contenant du desmostérol est d'abord acétylé par l'anhydride acétique dans la pyridine. Ensuite, on dissout 5 g du mélange d'acétate obtenu dans 200 ml d'acide acétique pur cristallisable. On introduit un barreau aimanté et la solution est portée aux environs de 45°C à l'aide d'un bain-marie. On ajoute alors 1,26 g d'iodate de potassium KIO₃ (5,9 mmoles). Ensuite, par petites fractions et en agitant magnétiquement, on ajoute 1,50 g d'iode I₂ (5,9 mmoles) préalablement pulvérisé à l'aide d'un mortier et le mélange est maintenu aux environs de 45°C pendant 2 h sous agitation. On ajoute alors 1,18 g d'acétate de potassium CH₃COOK (11,8 mmoles) et le mélange est porté à 80°C en bain-marie pendant 2 h. Après refroidissement, on ajoute 600 ml d'une solution aqueuse de thiosulfate de sodium à 1% et le produit qui précipite est extrait trois fois à l'éther diéthylique. L'extrait éthéré est 1°) lavé par une solution aqueuse de bicarbonate de sodium à 20% jusqu'à l'arrêt de l'effervescence, 2°) lavé à l'eau jusqu'à pH neutre des eaux de lavage, 3°) séché sur sulfate de sodium, filtré et évaporé à sec.

Le résidu obtenu (6 g) est séparé par chromatographie sur colonne de 150 g de silice. Le mélange hexane-éther éthylique: 99/1 élue le reste d'acétates de départ (1 g). Le mélange hexane-éther éthylique: 70/30 élue le diacétoxy-3β,24 hydroxy-25 cholestène-5 (3 g) qui est ensuite hydrolysé en dihydroxy 24,25 cholestérol suivant le procédé décrit précédemment.

Exemple 3

Le desmostérol est d'abord acétylé par l'anhydride acétique dans la pyridine. Ensuite, on dissout 500 mg d'acétyl-desmostérol (1,17

3

mmole) dans 20 ml d'acide acétique pur cristallisable. On introduit un barreau aimanté et la solution est portée aux environs de 45°C à l'aide d'un bain-marie. On ajoute alors 126 mg d'iodate de potassium KIO$_3$ (0,59 mmole). Ensuite, par petites fractions et en agitant magnétiquement, on ajoute 150 mg d'iode I$_2$ (0,59 mmole) préalablement pulvérisé à l'aide d'un mortier et le mélange est maintenu aux environs de 45°C pendant 8 h sous agitation. Le mélange réactionnel est ensuite traité suivant le processus décrit dans le procédé de base, à savoir refroidissement, addition de solution de thiosulfate de sodium, extraction du produit à l'éther, lavage au bicarbonate de sodium à 20%, lavage à l'eau, séchage sur sulfate de sodium, filtration, évaporation à sec, chromatographie sur colonne de 20 g de silice. On obtient 531 mg de diacétoxy-3$\beta$,24 hydroxy-25 cholestène-5. Rendement: 90%.

Le diacétoxy-3$\beta$,24 hydroxy-25 cholestène-5 ainsi obtenu est ensuite hydrolysé en dihydroxy-24,25 cholestérol suivant le processus décrit précédemment.

Exemple 4

Le mélange de stérols contenant du desmostérol est d'abord acétylé par l'anhydride acétique dans la pyridine. Ensuite, on dissout 5 g du mélange d'acétates obtenu dans 200 ml d'acide acétique pur cristallisable. On introduit un barreau aimanté et la solution est portée aux environs de 45°C à l'aide d'un bain-marie. On ajoute alors 1,26 g d'iodate de potassium KIO$_3$ (5,9 mmoles). Ensuite, par petites fractions et en agitant magnétiquement, on ajoute 1,50 g d'iode I$_2$ (5,9 mmoles) préalablement pulvérisé à l'aide d'un mortier et le mélange est maintenu aux environs de 45°C pendant 8 h sous agitation. Le mélange réactionnel est ensuite traité suivant le processus décrit dans le procédé de base, à savoir refroidissement, addition de solution de thiosulfate de sodium, extraction du produit à l'éther, lavage au bicarbonate de sodium à 20%, lavage à l'eau, séchage sur sulfate de sodium, filtration, évaporation à sec, chromatographie sur colonne de 150 g de silice. On obtient 3 g de diacétoxy-3$\beta$,24 hydroxy-25 cholestène-5 qui est ensuite hydrolysé en dihydroxy-24,25 cholestérol suivant le processus décrit précédemment.

Exemple 5

600 mg du résidu obtenu par évaporation à sec de l'éther éthylique au cours de la réaction sur le desmostérol pur suivant le procédé de base ou suivant la variante 1 de notre méthode sout dissous à chaud dans l'hexane. Au refroidissement à la température de la salle, le diacétoxy-3$\beta$,24 hydroxy-25 cholestène-5 cristallise et est recueilli par filtration, le filtrat obtenu est concentré et l'opération ci-dessus est recommencée une deuxième, puis une troisième fois. On obtient en somme 500 mg de

diacétoxy-3$\beta$,24 hydroxy-25 cholestène-5, Rendement: 87%.

Le diacétoxy-3$\beta$,24 hydroxy-25 cholestène-5 ainsi obtenu est ensuite hydrolysé en dihydroxy-24,25 cholestérol suivant le processus décrit précédemment.

Exemple 6

5 g du résidu obtenu par évaporation à sec de l'éther éthylique au cours de la réaction sur un mélange de stérols contenant du desmostérol suivant le procédé de base ou suivant la variante 1 de notre méthode sont dissous à chaud dans l'hexane. Au refroidissement à la température de la salle, le diacétoxy-3$\beta$,24 hydroxy-25 cholestène-5 cristalise et est recueilli per filtration; le filtrat obtenu est évaporé à sec et le résidu est dissous à chaud dans l'acétone. Au refroidissement, les stérols (sous forme d'acétates) qui n'avaient pas réagi au cours de la réaction cristallisent et sont recueillis par filtration; le filtrat obtenu est évaporé à sec et le résidu est repris à chaud dans l'hexane. Au refroidissement, une autre quantité de diacétoxy 3$\beta$,24 hydroxy-25 cholestène-5 cristallise. On obtient en somme 2,3 g de diacétoxy-3$\beta$,24 hydroxy-25 cholestène-5 qui est ensuite hydrolysé en dihydroxy-24,25 cholestérol suivant le processus décrit précédemment.

On donne ci-dessous quelques caractéristiques analytiques des composés obtenus (diacétoxy-3$\beta$,24 hydroxy-25 cholestène-5 et dihydroxy-24,25 cholestérol). Les données entre parenthèses correspondent aux composés obtenus suivant le procédé de base de notre méthode sur le mélange de stérols des graines de Funtumia elastica, tandis que les autres correspondent aux composés obtenus suivant le procédé de base de notre méthode sur le desmostérol pur.

Diacétoxy-3$\beta$,24 hydroxy-25 cholesténe-5
F.=162—163°C (hexane, tube capillaire)
    (164—165°C)
IR(KBr): 3475 cm$^{-1}$ (OH); 1740 cm$^{-1}$(acétates)
    (3470 cm$^{-1}$)      (1740 cm$^{-1}$)
RMN: $\delta$ ppm (CDL$_3$):
    0,67 (s,CH$_3$-18); 0,94 (d,CH$_3$-21);
    (0,67)          (0,94)
    1,02 (s,CH$_3$-19); 1,18 (s,CH$_3$-26, 27);
    (1,00)          (1,17)
    2,03 (s,Ac-3);    2,12 (s,Ac-24);
    (1,97)          (2,05)
    4,67 (m,H-3);     5,37 (m,H-6)
    (4,63)          (5,35)

Spectre de masse: m/e: 382 (100%, M$^+$-2AcOH); m/e: 442 (11%, M$^+$-AcOH)

Dihdroxy-24,25 cholestérol
F.=198—199°C (acétone, tube capillaire)
    (200—201°C)
IR(KBr): 3400 cm$^{-1}$ (OH)
    (3340 cm$^{-1}$)

RMN: $\delta$ ppm (ClCl$_3$): 0,70 (s,CH$_3$-18);
(pyridine)          (0,68)
   0,94 (d,CH$_3$-21); 1,04 (s,CH$_3$-19);
(0,97)                   (1,07)
   1,21 et 1,24 (s,CH$_3$-26 et 27);
(1,2 et 1,51)
   3,60 (m,H-3); 4,80 (t,H-24);
(3,64)              (4,84)
   5,34 (m,H-6)
(5,39)

Les composés obtenus suivant les autres variantes ont été à tout point de vue identiques aux précédents.

## Revendications

1. Procédé de préparation du dihydroxy-24, 25 cholestérol, caractérisé en ce que l'on soumet du desmostérol pur ou en mélange avec d'autres stérols naturels à une réaction d'acétylation, puis on traite le mélange réactionnel obtenu dans l'acide acétique par l'iode en présence d'iodate de potassium, puis l'iode en excès est détruit, que l'on extrait le produit réactionnel et évapore à sec la solution organique, que l'on soumet le résidu obtenu à une chromatographie ou à une cristallisation fractionnée et enfin que l'on hydrolyse le diacétoxy,3$\beta$,24 hydroxy-25 cholestène-5 par une solution alcoolique basique.

2. Procédé selon la revendication 1, caractérisé en ce que la réaction d'acétylation est réalisée par de l'anhydride acétique en milieu pyridine.

3. Procédé selon l'une des revendications 1 et 2, caractérisé en ce que le traitement par l'iode, en présence d'iodate de potassium, est complété par un traitement avec un acétate alcalin tel que l'acétate de potassium.

4. Procédé selon l'une des revendications 1, 2 et 3, caractérisé en ce que la destruction de l'excès d'iode est réalisée avec du thiosulfate de sodium.

5. Procédé selon l'une des revendications 1, 2, 3 et 4, caractérisé en ce que l'extrait contenant le diacétoxy-3$\beta$,24 hydroxy-25 cholestène-5 est lavé à l'aide de bicarbonate jusqu'à pH neutre, déshydraté, puis filtré et évaporé à sec.

## Patentansprüche

1. Verfahren zur Herstellung von Dihydroxy-24,25-cholesterin, dadurch gekennzeichnet, daß Desmosterol, rein oder in Mischung mit anderen natürlichen Sterinen, einer Acetylierungsreaktion unterworfen, die erhaltene Reaktionsmischung sodann in Essigsäure mit Jod in Gegenwart von Kaliumjodat behandelt, sodann das überschüssige Jod entfernt, das Reaktionsprodukt extrahiert und die organische Lösung zur Trockne eingedampft wird, daß der erhaltene Rückstand einer Chromatografie oder einer fraktionierten Kristallisation unterworfen und schließlich das Diacetoxy-3$\beta$,24-hydroxy-25-cholesten-5 mittels einer basischen Alkohollösung hydrolysiert wird.

2. Verfahren nach Anspruch 1, dadurch gekennzeichnet, daß die Acetylierungsreaktion mittels Essigsäureanhydrid im Pyridin-Medium durchgeführt wird.

3. Verfahren nach einem der Ansprüche 1 und 2, dadurch gekennzeichnet, daß die Jodbehandlung in Gegenwart von Kaliumjodat durch eine Behandlung mit einem Alkalimetallacetat, wie Kaliumacetat, vervollständigt wird.

4. Verfahren nach einem der Ansprüche 1, 2 und 3, dadurch gekennzeichnet, daß die Entfernung des überschüssigen Jods mit Natriumthiosulfat erfolgt.

5. Verfahren nach einem der Ansprüche 1, 2, 3 und 4, dadurch gekennzeichnet, daß der das Diacetoxy-3$\beta$,24-hydroxy-25-cholesten-5 enthaltende Extrakt mit Bicarbonatlösung bis zu einem pH-Wert im Neutralbereich gewaschen, getrocknet, anschließend filtriert und zur Trockne eingedampft wird.

## Claims

1. Process for the preparation of 24,25-dihydroxy cholesterol, characterised in that desmoterol in the pure state or mixed with other natural sterols is subjected to a reaction of acetylation, then the resulting reaction mixture is treated in acetic acid with iodine in the presence of potassium iodate, and the excess of iodine is destroyed, in that the reaction product is extracted and the organic solution is dry-evaporated, in that the resulting residue is subjected to a chromatography or to a fractional crystallization and finally in that the 3$\beta$,24-diacetoxy-25-hydroxy-5-cholestane is hydrolyzed by a basic alcoholic solution.

2. Process according to claim 1, characterised in that the acetylation reaction is caused by acetic anhydride in pyridine medium.

3. Process according to one of claims 1 and 2, characterized in that the treatment with iodine, in the presence of potassium iodate, is completed by a treatment with an alkaline acetate such as potassium acetate.

4. Process according to one of claims 1, 2 and 3, characterised in that the destruction of the excess of iodine is caused by sodium thiosulphate.

5. Process according to one of claims 1, 2, 3 and 4, characterised in that the extract containing the 3$\beta$,24-diacetoxy-25-hydroxy-5-cholestene is washed with bicarbonate until a neutral pH is reached, when dehydrated and filtered and dry-evaporated.